**Europäisches Patentamt**

**European Patent Office**

**Office européen des brevets**

(19)

(11) Veröffentlichungsnummer : **0 029 591**
**B1**

(12) **EUROPÄISCHE PATENTSCHRIFT**

(45) Veröffentlichungstag der Patentschrift :
22.09.82

(21) Anmeldenummer : 80107265.3

(22) Anmeldetag : 21.11.80

(51) Int. Cl.³ : **C 07 C 51/58, C 07 C 53/48,
C 07 C 69/63**

(54) Verfahren zur Herstellung von Trifluoracetylchlorid.

(30) Priorität : 24.11.79 DE 2947376

(43) Veröffentlichungstag der Anmeldung :
03.06.81 (Patentblatt 81/22)

(45) Bekanntmachung des Hinweises auf die Patenterteilung : 22.09.82 Patentblatt 82/38

(84) Benannte Vertragsstaaten :
AT BE CH DE FR GB IT LI NL

(56) Entgegenhaltungen :
JOURNAL OF THE CHEMICAL SOCIETY, Part I,
1959, Seiten 387-396 The Chemical Society
London, G.B. R.N. HASZELDINE et al. :
« Oxidation of polyhalogeno-compounds. Part II,
Photolysis and photochemical exidation of some
chlorofluoro-ethanes » Seiten 387-396

(73) Patentinhaber : Kali-Chemie Aktiengesellschaft
Postfach 220 Hans-Böckler-Allee 20
D-3000 Hannover 1 (DE)

(72) Erfinder : Rudolph, Werner, Dipl.-Chem., Dr.
Oderstrasse 38
D-3000 Hannover 73 (DE)
Erfinder : Fernschild, Günter
Am Asphaltberge 8
D-3000 Hannover 91 (DE)
Erfinder : Hirsch, Wolfgang, Dipl.-Chem., Dr.
Humboldtstrasse 26
D-3300 Braunschweig (DE)

## 0 029 591

### Verfahren zur Herstellung von Trifluoracetylchlorid

Die Erfindung betrifft ein Verfahren zur Herstellung von Trifluoracetylchlorid durch Umsetzung von 1.1.1-Trifluor-2.2.2-trichloräthan mit Schwefeltrioxid in Gegenwart von Quecksilbersalzen.

Ein solches Verfahren ist aus der US-PS 3 160 659 bekannt. Danach kann Perfluoracetylchlorid erhalten werden, wenn in Trifluortrichloräthan, welchem Quecksilbersulfate als Katalysatoren zugegeben wurden, bei der Rückflußtemperatur langsam Oleum oder $SO_3$ eingeführt wird und diese Mischung anschließend längere Zeit unter Rückfluß am Sieden gehalten wird. Nach einigen Stunden beginnt dann das Abdestillieren des Trifluoracetylchlorids. Zur vollständigen Umsetzung werden aber mehrere Tage benötigt.

Auch die Herstellung von fluorhaltigen Perhalogencarbonsäuren durch Solvolyse bereitet Schwierigkeiten. Relativ einfach gelingt die Solvolyse von perchlorierten aliphatischen Verbindungen, die eine oder mehrere $CCl_3$-Gruppen enthalten. Sie können durch mäßig konzentrierte Schwefelsäure zu Carbonsäuren mit einer oder mehreren Carboxylgruppen umgewandelt werden, wie H. Henecka in Houben Weyl, Methoden der organischen Chemie, Bd. 8, S. 427, beispielsweise ausführt. Stehen in Hachbarschaft zur endständigen $CCl_3$-Gruppe ein oder mehrere Fluoratome, so findet eine Carbonsäurebildung nur statt, wenn freies $SO_3$ enthaltende Schwefelsäure oder $SO_3$ eingesetzt werden. Durch Variation des $SO_3$-Gehaltes der Schwefelsäure, der $SO_3$-Menge zum Halogenalkan, der Reaktionstemperatur oder Reaktionsdauer wurde schon versucht, die Verfahren so zu verbessern, daß eine technische Durchführung möglich ist. Die Umsetzung wird teils in Abwesenheit, teils in Gegenwart von katalysatoren, wie Quecksilbersulfaten, durchgeführt (USA-Patentschriften 2 396 076 und 3 102 139). Die Reaktionsgeschwindigkeiten der nur diskontinuierlich durchführbaren Verfahren sind bisher aber immer unbefriedigend. Ein weiterer Nachteil dieser Verfahren ist die oft umständliche Gewinnung der Säure aus dem Reaktionsgemisch. Im allgemeinen wird dieses mit Wasser verdünnt und die Säure durch langwierige und kostspielige Extraktionsprozesse gewonnen. Oft tritt Zersetzung der Säure bei der Aufarbeitung ein, so daß auch hierdurch noch eine Ausbeuteminderung erfolgt.

Gemäß DE-PS 19 17 630 werden fluorhaltige Perhalogencarbonsäurefluoride oder -chloride aus Fluorchloralkanen durch Umsetzung mit $SO_3$ in Anwesenheit von katalytischen Mengen von Quecksilbersulfaten hergestellt. Dieses Verfahren eignet sich für einen kontinuierlichen Betrieb, wobei brauchbare, aber noch nicht optimale Raum-Zeit-Ausbeuten erreicht werden.

Der Erfindung liegt die Aufgabe zugrunde, die Nachteile des Standes der Technik zu überwinden. Überraschenderweise kann eine Beschleunigung der Reaktion erreicht werden, insbesondere beim diskontinuierlichen Betrieb, wenn gattungsgemäß 1.1.1-Trifluor-2.2.2-trichloräthan mit Schwefeltrioxid in Gegenwart von Quecksilbersalzen umgesetzt wird, wobei man die Umsetzung erfindungsgemäß zusätzlich in Gegenwart von Borhalogenid $BX_3$ ($X = F$, Cl, Br, J) und/oder halogensulfonsäure $HSO_3Y$ ($Y = F$, Cl, Br, J) ausführt. Sowohl für X als auch für Y sind Chlor oder Brom das bevorzugte Halogen.

Eine Variante des erfindungsgemäßen Verfahrens ist, die Reaktion nur in zusätzlicher Gegenwart von Borhalogenid auszuführen.

Es ist zwar aus der US-PS 3 102 139 bekannt, u.a. $CF_3CCl_3$ in Gegenwart von Quecksilbersalzen mit stabilisiertem $SO_3$ umzusetzen. Das als Stabilisator genannte Boroxid vermag in diesem System aber ebensowenig zur Verbesserung der Reaktion beizutragen, wie das ausdrücklich als einsetzbarer Katalysator bezeichnete Aluminiumtrichlorid. Es war daher nicht naheliegend, Versuche mit Borhalogeniden in erfindungsgemäßer Weise vorzunehmen.

Eine bevorzugte Variante des erfindungsgemäßen Verfahrens besteht darin, die Reaktion nur in zusätzlicher Gegenwart von Halogensulfonsäure auszuführen. Diese Verfahrensweise arbeitet sogar entgegengesetzt zu der in der US-PS 3 102 139 vermittelten Lehre, wonach die Umsetzung des Systems fluorhalogenalkan/Schwefeltrioxid/Katalysator in Abwesenheit von Verbindungen erfolgen muß, die aktiven (Zerewitinoff) Wasserstoff enthalten.

Eine besonders bevorzugte Variante des erfindungsgemäßen Verfahrens besteht darin, die Reaktion in zusätzlicher Gegenwart von Borhalogenid und Halogensulfonsäure auszuführen. Mit dieser Verfahrensvariante werden die besten Resultate erzielt.

In allen Fällen, in denen die Reaktion zusätzlich in Gegenwart von Borhalogenid abläuft, kann dieses als solches dem Reaktionsgemisch zugesetzt werden.

Es ist aber in einer anderen Variante auch möglich, das Borhalogenid aus einer geeigneten Borverbindung und einem Halogenierungsmittel in situ zu erzeugen.

Unter geeigneten Borverbindungen sind solche zu verstehen, die zusammen mit dem Halogenierungsmittel unter den vorgegebenen Reaktionsbedingungen Borhalogenid bilden. Besonders geeignete Borverbindungen sind sauerstoffhaltige Borverbindungen, wie z.B. Borsäure, Boroxid oder Natriumborat, wobei Natriumtetraborat besonders geeignet ist.

Als Halogenierungsmittel kommen solche in Frage, die unter den vorgegebenen Reaktionsbedingungen mit der Borverbindung Borhalogenid ergeben. Nach den vorliegenden Erkenntnissen ist Aluminiumtrichlorid nicht als Halogenierungsmittel wirksam. Es können Schwefelhalogenide, Schwefeloxyhalogenide, Phosphorhalogenide, Phosphoroxyhalogenide und/oder Halogensulfonsäuren verwendet werden. Letztere sind besonders bevorzugt, insbesondere auch deshalb, weil in der besonders

2

bevorzugten Variante des erfindungsgemäßen Verfahrens die Reaktion sowohl in Gegenwart von Borhalogenid als auch von Halogensulfonsäure abläuft. Setzt man also zur in situ-Bildung des Borhalogenids als Halogenierungsmittel einen — bezogen auf zu halogenierende Borverbindung — Stöchiometrischen Überschuß an Halogensulfonsäure ein, so kommt man automatisch zu der besonders bevorzugten Verfahrensvariante.

In allen Fällen, in denen die Reaktion zusätzlich in Gegenwart von Halogensulfonsäure abläuft, kann man diese als solche dem Reaktionsgemisch zusetzen. Es ist aber auch möglich, die Halogensulfonsäure in an sich bekannter Weise in situ im Reaktionsgemisch zu erzeugen. Besonders geeignet zur in situ Herstellung von Halogensulfonsäure ist die Umsetzung von $SO_3$ mit Halogenwasserstoff.

Die Umsetzung von Trifluor-trichloräthan mit $SO_3$ zum Trifluoracetylchlorid — gemäß dem erfindungsgemäßen Verfahren — läßt sich bei Atmosphärendruck im Temperaturbereich von 0 bis 120 °C durchführen, wobei der bevorzugte Temperaturbereich 20° bis 80° ist. Es ist aber durchaus möglich, die Reaktion bei Temperaturen oberhalb 120 °C und/oder bei Drücken oberhalb Atmosphärendruck ablaufen zu lassen, jedoch erfordert das einen zusätzlichen technischen Aufwand.

Das Verfahren kann chargenweise, halbkontinuierlich oder kontinuierlich betrieben werden. Bei chargenweisem Betrieb kann das Borhalogenid und/oder die Halogensulfonsäure bzw die zur Bildung dieser Verbindung bzw. Verbindungen eingesetzten Ausgangsverbindungen zu der im Reaktionsgefäß vorgelegten Reaktionsmischung ($CF_3CCl_3 + SO_3 +$ Quecksilbersalz) nachträglich zugesetzt werden. Die Reihenfolge der Zugaben spielt aber keine wesentliche Rolle, so daß sie auch abgeändert werden kann. Die Reaktionsgeschwindigkeit nimmt im Verlauf der Reaktion durch den Verbrauch der Ausgangskomponenten ab. Um eine quantitative Umsetzung des $CF_3CCl_3$ zu erreichen, wird $SO_3$ im Überschuß verwendet, wobei günstige Raum-Zeit-Ausbeuten erreicht werden, wenn 1 bis 10 Mol, vorzugsweise 1 bis 5 Mol $SO_3$ pro Mol $CF_3$-$CCl_3$ zur Verfügung stehen. Ein höherer $SO_3$-Gehalt im Reaktionsgemisch hat keinen wesentlichen Einfluß auf die Reaktionsgeschwindigkeit und die Ausbeute.

Ein Einfluß der Menge an Borhalogenid und/oder Halogensulfonsäure — hinsichtlich der Ausbeute und der Reaktionsgeschwindigkeit — auf die Reaktion ist begrenzt feststellbar. Ein günstiger Reaktionsverlauf wird bei Bor-Gehalten von 1 bis 30 Mol-%, vorzugsweise 1 bis 10 Mol-%, bezogen auf eingesetztes Fluorchloralkan erreicht. Die Mengen an Halogensulfonsäure betragen vorteilhafterweise 3 bis 140 Mol-%, insbesondere 3 bis 50 Mol-%, bezogen auf eingesetztes fluorchloralkan. Höhere Gehalte an Borhalogenid und/oder Halogensulfonsäure verbessern die Reaktionsgeschwindigkeit nur im geringen Maße.

Das bei der Reaktion gebildete Trifluoracetylchlorid wird entweder direkt quantitativ erfaßt oder aber zu anderen Trifluoracetylverbindungen in an sich bekannter Weise umgesetzt. So kann es z.B. in Äthanol eingeleitet und zu Trifluoressigsäureäthylester umgesetzt werden. Anstelle von Äthanol können auch andere aliphatische oder aromatische Alkohole eingesetzt werden. Ebenso sind auch Umsetzungen mit anderen Verbindungen möglich, die bekanntermaßen mit Säurechloriden eine Reaktion eingehen können, wie z.B. Ammoniak, Amine, Wasser, Keten etc. Auf diese Weise gelangt man zu wertvollen Trifluoracetylverbindungen, die auf vielen Gebieten der Technik verwendet werden können, z.B. auf dem Arzneimitteloder Pflanzenschutzsektor.

## Beispiele

Die in den Beispielen 1 bis 7 erhaltenen Ausbeuten an Trifluoracetylchlorid in Mol.-%, bezogen auf Trifluortrichloräthan werden in Tabelle 1 in Abhängigkeit von der Reaktionszeit angegeben.

## Beispiel 1

In einem 250 ml Dreihalskolben aus Glas, mit aufgesetztem Rückflußkühler, der auf 0 °C thermostatisiert wird, werden 1 g $HgSO_4$ und 1 g $H_2SO_4$ vorgelegt. Zu diesem Gemisch wird eine Lösung von 93,7 g (0,5 Mol) $CF_3$-$CCl_3$ und 162 g (2,0 Mol) $SO_3$ eingefüllt. Nach einer weiteren Zugabe von 17,5 g (0,18 Mol) $HSO_3F$ wird das Reaktionsgemisch bei Raumtemperatur gerührt und in diese Lösung 2 g $BF_3$ (0,03 Mol) eingeleitet. Anschließend wird der Kolbeninhalt erwärmt, wobei ab 30 °C eine lebhafte $CF_3$-$COCl$-Entwicklung einsetzt. Das Trifluoracetylchlorid durchströmt den Rückflußkühler, passiert einen $H_2SO_4$-Wäscher, um mitgerissenes $SO_3$ abzutrennen und wird in zwei nachfolgenden Kühlfallen bei —78 °C auskondensiert. Im Verlauf der Reaktion steigt die Temperatur im Kolben von 30° auf 59 °C an.

## Beispiel 2

In die in Beispiel 1 beschriebene Versuchsapparatur werden 93,7 g $CF_3CCl_3$ eingesetzt und 2 g $HgSO_4$ zugegeben. Die Aufschlämmung wird bei 20 °C gerührt. Nachfolgend wird eine Lösung von 162,0 g $SO_3$, 17,5 g $HSO_3F$ und 2 g $BF_3$ eingetropft. Eine intensive Reaktion setzt bei 38 °C ein. Die Endtemperatur im Reaktionsgefäß betrug 56 °C.

## Beispiel 3

In den Kolben der im Beispiel 1 beschriebenen Versuchsapparatur werden 4 g $H_3BO_3$ und 2 g $HgSO_4$

3

eingewogen. Dazu wird aus einem Tropftrichter eine Mischung von 93,7 g $CF_3CCl_3$, 162 g $SO_3$ und 17,5 g $HSO_3Cl$ gegeben. Das Reaktionsgemisch erwärmt sich unter starker Gasentwicklung auf 35 °C.

Beispiel 4

In der in Beispiel 1 beschriebenen Versuchsapparatur werden 11 g $HgSO_4$, 1 g $B_2O_3$ und 93,7 g $CF_3CCl_3$ vorgelegt. Aus einem Tropftrichter wird eine Lösung von 162 g $SO_3$, 20 g $HSO_3Cl$ und 1 g $BF_3$ zugesetzt. Dabei steigt die Temperatur im Reaktionsgefäß auf 37 °C an.

Beispiel 5

In die in Beispiel 1 beschriebene Versuchsapparatur werden 2 g $HgSO_4$ und 10 g $BBr_3$ vorgelegt. Dazu wird eine Lösung von 93,7 g $CF_3CCl_3$, 162 g $SO_3$ und 4 g $H_2SO_4$ eingetropft. Das den Rückflußkühler verlassende Trifluoracetylchlorid wird durch eine Waschflasche mit $H_2SO_4$ geleitet und anschließend in ein Reaktionsgefäß mit Äthanol eingespeist. Hierbei entsteht Trifluoressigsäureäthylester.

Beispiel 6

In die in Beispiel 1 beschriebene Versuchsapparatur wird ein Gemisch von 1 g $HgSO_4$, 1 g $Hg_2SO_4$, 93,7 g $CF_3CCl_3$, 162 g $SO_3$ und 17,5 g $HSO_3Cl$ vorgelegt. Anschließend wird unter Rühren das Reaktionsgemisch auf 38 °C erwärmt. Im Verlauf der Reaktion steigt die Reaktionstemperatur auf 67 °C. Nach 12 h hört die Gasentwicklung auf.

Beispiel 7

In den Kolben der in Beispiel 1 beschriebenen Versuchsapparatur werden 5 g $Na_2B_4O_7 \times 10 \, H_2O$ und 4 g $HgSO_4$ eingewogen. Aus einem Tropftrichter wird ein Gemisch von 94,7 g $CF_3CCl_3$, 162 g $SO_3$ und 20 g $HSO_3Cl$ gegeben, wobei es unter Erwärmung zu einer kräftigen Gasentwicklung kommt. Die sich anfangs auf 32 °C einstellende Temperatur im Kolben erhöht sich nach sechsstündiger Erwärmung am Rückfluß auf 74 °C.

Tabelle 1

Ausbeute an Trifluoracetylchlorid

| Reaktionszeit [h] | Beispiel 1 | | Beispiel 2 | | Beispiel 3 | | Ausbeute Beispiel 4 | | Beispiel 5 | | Beispiel 6 | | Beispiel 7 | |
|---|---|---|---|---|---|---|---|---|---|---|---|---|---|---|
| | g | % | g | % | g | % | g | % | g | % | g | % | g | % |
| 1 | 16,8 | 25,0 | 37,1 | 55,4 | 35,0 | 52,2 | 35,0 | 52,2 | | | | | | |
| 2 | 34,4 | 52,9 | 42,5 | 63,4 | 47,6 | 71,1 | 47,3 | 70,6 | | | | | 50,2 | 74,9 |
| 3 | 39,1 | 58,4 | 44,9 | 67,0 | 53,7 | 80,2 | 53,4 | 79,7 | | | | | | |
| 4 | 42,3 | 63,2 | 47,0 | 70,1 | 58,1 | 86,7 | 55,9 | 83,4 | | | | | 63,1 | 94,2 |
| 6 | 46,0 | 68,7 | 50,3 | 75,0 | 59,6 | 89,0 | 58,0 | 86,6 | | | | | 63,9 | 95,4 |
| 8 | 47,6 | 71,1 | 53,5 | 79,8 | 60,2 | 89,9 | | | | | | | | |
| 12 | | | | | | | | | 115,8 | 81,0 | 52,3 | 78,0 | | |

Beispiel 8 (nicht erfindungsgemäß)

In der in Beispiel 1 beschriebenen Versuchsapparatur wird ein Gemisch von 94,7 g $CF_3CCl_3$, 162 g $SO_3$, 5 g fein gepulvertes, wasserfreies Aluminiumchlorid und 2 g $HgSO_4$ über einen Zeitraum von 4 h erwärmt. Im Kolben stellt sich bei gutem Rückfluß eine gleichbleibende Temperatur von 38 °C ein. Eine Gasentwicklung findet nicht statt, Trifluoracetylchlorid wird nicht erhalten.

**Ansprüche**

1. Verfahren zur Herstellung von Trifluoracetylchlorid ($CF_3COCl$), durch Umsetzung von 1.1.1-Trifluor-2.2.2-trichloräthan ($CF_3CCl_3$) mit Schwefeltrioxid in Gegenwart von Quecksilbersalzen, dadurch gekennzeichnet, daß man die Reaktion zusätzlich in Gegenwart von Borhalogenid $BX_3$ (X = F, Cl, Br, J) und/oder Halogensulfonsäure $HSO_3Y$ (Y = F, Cl, Br, J) ausführt.

2. Verfahren nach Anspruch 1, dadurch gekennzeichnet, daß man die Umsetzung in Gegenwart von Borhalogenid ausführt.

4

3. Verfahren nach Anspruch 1, dadurch gekennzeichnet, daß man die Umsetzung in Gegenwart von Borhalogenid und Halogensulfonsäure ausführt.

4. Verfahren nach einem der Ansprüche 1 bis 3, dadurch gekennzeichnet, daß man das Borhalogenid als solches zum Reaktionsgemisch zusetzt.

5. Verfahren nach einem der Ansprüche 1 bis 3, dadurch gekennzeichnet, daß man das Borhalogenid in situ aus einer Borverbindung und einem Halogenierungsmittel herstellt.

6. Verfahren nach Anspruch 5, dadurch gekennzeichnet, daß man als Borverbindung Borsäure, Bortrioxid und/oder Natriumborat, vorzugsweise Natriumtetraborat, verwendet.

7. Verfahren nach Anspruch 5 oder 6, dadurch gekennzeichnet, daß man als Halogenierungsmittel eine Verbindung aus der Gruppe Schwefelhalogenid, Schwefeloxyhalogenid, Phosphorhalogenid, Phosphoroxyhalogenid, Halogensulfonsäure und/oder deren Gemische verwendet.

8. Verfahren nach Anspruch 7, dadurch gekennzeichnet, daß man Halogensulfonsäure verwendet.

9. Verfahren nach einem der Ansprüche 1 bis 8, dadurch gekennzeichnet, daß die eingesetzte Menge an Borhalogenid oder Borverbindung 1 bis 30 Mol-%, vorzugsweise 1 bis 10 Mol-%, Bor beträgt, bezogen auf die eingesetzte Menge an Trifluor-trichloräthan.

10. Verfahren nach Anspruch 1, dadurch gekennzeichnet, daß man die Umsetzung in Gegenwart von Halogensulfonsäure ausführt.

11. Verfahren nach einem der Ansprüche 1 oder 3 bis 10, dadurch gekennzeichnet, daß man die Halogensulfonsäure als solche zum Reaktionsgemisch zusetzt.

12. Verfahren nach einem der Ansprüche 1 oder 3 bis 10, dadurch gekennzeichnet, daß man die Halogensulfonsäure in situ herstellt.

13. Verfahren nach einem der Ansprüche 1 oder 3 bis 12, dadurch gekennzeichnet, daß die eingesetzte Menge an Halogensulfonsäure 3 bis 140 Mol-%, vorzugsweise 3 bis 50 Mol-% beträgt, bezogen auf die eingesetzte Menge an Trifluor-trichloräthan.

14. Verfahren nach einem der vorangehenden Ansprüche, dadurch gekennzeichnet, daß die eingesetzte Menge von $SO_3$ zwischen 1 und 10 Mol, vorzugsweise zwischen 1 und 5 Mol, pro Mol eingesetztes Trifluortrichloräthan beträgt.

15. Verfahren nach einem der vorangehenden Ansprüche, dadurch gekennzeichnet, daß die Reaktionstemperatur bei Atmosphärendruck zwischen 0 und 120 °C, vorzugsweise zwischen 20 und 80 °C liegt.


## Claims

1. Process for the production of trifluoroacetyl chloride ($CF_3COCl$), by the reaction of 1.1.1-trifluoro-2.2.2-trichloroethane ($CF_3CCl_3$) with sulphur trioxide in the presence of mercury salts, characterised in that the reaction is, in addition, carried out in the presence of a boron halide $BX_3$ (X = F, Cl, Br, I) and/or a halosulphonic acid $HSO_3Y$ (Y = F, Cl, Br, I).

2. Process according to Claim 1, characterised in that the reaction is carried out in the presence of a boron halide.

3. Process according to Claim 1, characterised in that the reaction is carried out in the presence of a boron halide and a halosulphonic acid.

4. Process according to one of Claims 1 to 3, characterised in that the boron halide is added as such to the reaction mixture.

5. Process according to one of Claims 1 to 3, characterised in that the boron halide is produced in situ from a boron compund and a halogenating agent.

6. Process according to Claim 5, characterised in that the boron compounds is boric acid, boric oxide and/or sodium borate, preferably sodium tetraborate.

7. Process according to Claim 5 or 6, characterised in that the halogenating agent is a compound of the group sulphur halides, sulphur oxyhalides, phosphorus halides, phosphorus oxyhalides, halosulphonic acids and/or mixtures thereof.

8. Process according to Claim 7, characterised in that a halosulphonic acid is used.

9. Process according to one of Claims 1 to 8, characterised in that the amount of boron halide or boron compound used is 1 to 30 mol-%, preferably 1 to 10 mol-% boron, based on the amount of trifluorotrichloroethane used.

10. Process according to Claim 1, characterised in that the reaction is carried out in the presence of a halosulphonic acid.

11. Process according to one of Claims 1 or 3 to 10, characterised in that the halosulphonic acid is added as such to the reaction mixture.

12. Process according to one of Claims 1 or 3 to 10, characterised in that the halosulphonic acid is produced in situ.

13. Process according to one of Claims 1 or 3 to 12, characterised in that the amount of halosulphonic acid used is 3 to 140 mol-%, preferably 3 to 50 mol-%, based on the amount of trifluorotrichloroethane used.

14. Process according to one of the preceding Claims, characterised in that the amount of $SO_3$ used

is between 1 and 10 mol, preferably between 1 and 5 mol, per mol of trifluorotrichloroethane used.

15. Process according to one of the preceding Claims, characterised in that the reaction temperature at atmospheric pressure is between 0 and 120 °C, preferably between 20 and 80 °C.

## Revendications

1. Procédé pour la préparation de chlorure de trifluoroacétyle (CF$_3$COCl) par réaction de 1,1,1-trifluoro-2,2,2-trichloroéthane (CF$_3$CCl$_3$) avec du trioxyde de soufre en présence de sels de mercure, caractérisé en ce qu'on conduit la réaction en présence en outre d'un halogénure de bore BX$_3$ (X = F, Cl, Br, I) et/ou d'un acide halogénosulfonique HSO$_3$Y (Y = F, Cl, Br, I).

2. Procédé selon la revendication 1, caractérisé en ce qu'on conduit la réaction en présence d'un halogénure de bore.

3. Procédé selon la revendication 1, caractérisé en ce qu'on conduit la réaction en présence d'un halogénure de bore et d'un acide halogénosulfonique.

4. Procédé selon l'une quelconque des revendications 1 à 3, caractérisé en ce qu'on ajoute l'halogénure de bore tel quel au mélange réactionnel.

5. Procédé selon l'une quelconque des revendications 1 à 3, caractérisé en ce qu'on prépare l'halogénure de bore in situ à partir d'un composé de bore et d'un agent d'halogénation.

6. Procédé selon la revendication 5, caractérisé en ce qu'on utilise comme composé de bore l'acide borique, le trioxyde de bore et/ou le borate de sodium, de préférence le tétraborate de sodium.

7. Procédé selon l'une des revendications 5 ou 6, caractérisé en ce qu'on utilise comme agent d'halogénation un composé choisi parmi un halogénure de soufre, un oxyhalogénure de soufre, un halogénure de phosphore, un oxyhalogénure de phosphore, un acide halogénosulfonique et/ou leurs mélanges.

8. Procédé selon la revendication 7, caractérisé en ce qu'on utilise un acide halogénosulfonique.

9. Procédé selon l'une quelconque des revendications 1 à 8, caractérisé en ce que la quantité d'halogénure de bore ou de composé de bore utilisée correspond à une teneur en bore de 1 à 30 moles %, de préférence de 1 à 10 moles %, par rapport à la quantité de trifluoro-trichloroéthane utilisée.

10. Procédé selon la revendication 1, caractérisé en ce qu'on conduit la réaction en présence d'un acide halogénosulfonique.

11. Procédé selon l'une quelconque des revendications 1 ou 3 à 10, caractérisé en ce qu'on ajoute l'acide halogénosulfonique tel quel au mélange réactionnel.

12. Procédé selon l'une quelconque des revendications 1 ou 3 à 10, caractérisé en ce qu'on prépare l'acide halogénosulfonique in situ.

13. Procédé selon l'une quelconque des revendications 1 ou 3 à 12, caractérisé en ce que la quantité d'acide halogénosulfonique utilisée atteint de 3 à 140 moles %, de préférence de 3 à 50 moles %, par rapport à la quantité de trifluoro-trichloroéthane ajoutée.

14. Procédé selon l'une quelconque des revendications précédentes, caractérisé en ce que la quantité de SO$_3$ utilisée est comprise entre 1 et 10 moles, de préférence entre 1 et 5 moles, par mole de trifluoro-trichloroéthane ajouté.

15. Procédé selon l'une quelconque des revendications précédentes, caractérisé en ce que la température de réaction à la pression atmosphérique est comprise entre 0 et 120 °C, de préférence entre 20 et 80 °C.